# EUROPÄISCHE PATENTSCHRIFT

(11) **EP 3 381 392 B1**
(45) Veröffentlichungstag und Bekanntmachung des Hinweises auf die Patenterteilung: **04.06.2025**
(21) Anmeldenummer: 18156310.7
(22) Anmeldetag: 12.02.2018
(51) Int. Cl.: A61B 18/12, A61B 18/14, A61B 18/00

(54) **GENERATOR ZUR VERSORGUNG EINES KOAGULATIONSINSTRUMENTS**
GENERATOR FOR SUPPLYING A COAGULATION INSTRUMENT
GÉNÉRATEUR PERMETTANT D'ALIMENTER UN INSTRUMENT DE COAGULATION

(30) Priorität: 29.03.2017 DE 102017106747
(43) Veröffentlichungstag der Anmeldung: 03.10.2018
(73) Patentinhaber: Erbe Elektromedizin GmbH, 72072 Tübingen (DE)
(72) Erfinder: Nold, Bernhard Tobias, 72070 Tübingen (DE); Neugebauer, Alexander, 72116 Mössingen (DE); Ederer, Michael, 70563 Stuttgart (DE); Fischer, Klaus, 72202 Nagold (DE); Enderle, Markus, 72070 Tübingen (DE); Wagenpfeil, Jay, 70197 Stuttgart (DE); Rex, Julia, 70569 Stuttgart (DE); Feuer, Ronny, 73733 Esslingen (DE); Sawodny, Oliver, 70186 Stuttgart (DE)
(74) Vertreter: Rüger Abel Patentanwälte PartGmbB

(56) Entgegenhaltungen:
- EP-A1- 1 082 944
- EP-A2- 0 558 317
- EP-A2- 0 978 259
- EP-A2- 2 767 249
- WO-A2-2015/184446
- US-A1- 2013 035 679
- C. THIEL ET AL: "Gewebedifferenzierende Merkmale in den elektrischen Signalen bei der HF-Chirurgie", BIOMEDIZINISCHE TECHNIK., vol. 41, no. s1, 1 January 1996 (1996-01-01), DE, pages 468 - 469, XP055465440, ISSN: 0013-5585, DOI: 10.1515/bmte.1996.41.s1.468

## Beschreibung

Die Erfindung betrifft einen Generator zur Versorgung eines Gewebefusionsinstruments, insbesondere eines Instruments zur Gefäßfusion.

Die chirurgische Anwendung von Koagulationsinstrumenten, insbesondere Gewebefusionsinstrumenten am Patienten erfolgt meist unter erheblichem Zeitdruck. Sind bei einem chirurgischen Eingriff viele Koagulationsmaßnahmen durchzuführen, insbesondere viele Gefäße zu verschließen und gegebenenfalls zu durchtrennen, kommt es darauf an, dass das Schließen der Gefä-ße in möglichst kurzer Zeit erfolgt. Es soll möglichst wenig umgebendes Gewebe geschädigt und koaguliert werden, um unerwünschte Läsionen zu minimieren. Andererseits muss der Verschluss sicher erfolgen, damit geschlossene und danach durchtrennte Gefäße sich nicht während oder nach der Operation öffnen und zu Blutungen führen.

Die Gefäßfusion erfolgt typischerweise zwischen zwei Branchen eines Fusionsinstruments, die mit hochfrequenter Koagulationsspannung beaufschlagt werden und ein zwischen ihnen gefasstes Gefäß zusammendrücken und mittels Stromdurchfluss erhitzen.

Ein solches Instrument und dazugehörige Koagulationsprozesse sind der US 8,216,223 B2 zu entnehmen. Dem Instrument ist eine Einrichtung zugeordnet, die zu Beginn eines Koagulationsprozesses einen Testimpuls an das Instrument gibt, um die Gewebeimpedanz zu erfassen. Zusätzlich oder alternativ kann das System zu Beginn der Behandlung Charakteristika des elektrochirurgischen Instruments bestimmen. Sodann bestimmt das System, ob eine Reaktion des Gewebes zu verzeichnen ist und ermittelt daraus eine gewünschte Impedanztrajektorie. Daraus ermittelt das System auf der Basis einer gewünschten Änderungsrate der Impedanz deren Zielwert. Das System überwacht dann die Einhaltung dieses gewünschten Impedanzverlaufs, wobei es die Temperatur, den Gewebetyp oder ähnliches erfasst. Zusätzlich kann das System die bei dem Versiegelungsprozess an das Gewebe gelieferte Energiemenge erfassen und die weitere Energielieferung für eine vorbestimmte Zeitspanne stoppen, wenn die Impedanz eine Schwelle übersteigt, die über dem Wert der anfänglichen Impedanz liegt. Wenn die Energiebeaufschlagung des Gewebes beendet ist, kann das System eine Abkühlzeit vorsehen. Die Abkühlzeit dient zur Verfestigung des Kollagens in dem versiegelten Gewebe, wobei als Abkühlzeit eine feste Zeitspanne oder eine adaptive Zeitspanne vorgesehen sein kann, die von Parametern abhängt, die mit dem Gewebefusionsprozess in Verbindung stehen. Nach Ablauf der Kühlzeitspanne ist der Versiegelungsprozess beendet. Das System kann aktive Kühlelemente enthalten, um die Kühlung zu beschleunigen, wie beispielsweise Heatpipes oder Peltier-Elemente.

Aus der WO 2015/184446 A2 ist ein Koagulationsinstrument bekannt, das zwischen Branchen einer Gewebezange gehaltenes Gewebe mittels Stromdurchfluss erhitzt. Dabei wird der Phasenwinkel des durch das Gewebe geschickten Wechselstroms erfasst, um das Gewebe zur Fusionierung in einem 2-Phasenzustand bei 100° C zu halten. Die US 5,827,271 veranschaulicht ebenfalls ein Gewebefusionsinstrument, bei dem Gefäße zwischen zwei bestromten Branchen zusammengedrückt und von Strom durchflossen und somit erhitzt werden, um das Gewebe bzw. Gefäß zu fusionieren. Nachdem die Fusion erfolgt ist, wird die Leistungsabgabe an das Instrument auf ein sehr niedriges Niveau gesenkt, um eine Abkühlung des Gewebes in der schnellstmöglichen Zeit zu erreichen. Alternativ kann eine sehr geringe Leistung von ungefähr 1 Watt an das Gewebe abgegeben werden, um den Stromkreis über das Gewebe weiterhin geschlossen zu halten. Eine derart geringe Leistungsabgabe verzögert den Abkühlvorgang nicht.

Es soll in dem koagulierten Gewebe eine möglichst homogene Struktur erzielt werden. Die Erzielung einer homogenen Gewebestruktur und zugleich einer kurzen Versiegelungszeit stehen in einem Zielkonflikt. Es ist Aufgabe der Erfindung, ein Konzept anzugeben, mit dem sich die Homogenität und damit die Sicherheit der Versiegelung verbessern und die Versiegelungszeit verkürzen lässt.

Diese Aufgabe wird mit einem Generator nach Anspruch 1 oder 4 gelöst.

Der erfindungsgemäße Generator erzeugt eine Koagulationsspannung zum Betrieb des Instruments zur Gewebefusion in mehreren Etappen. Der Generator ist dafür dazu eingerichtet, in einer ersten Etappe das Gewebe zunächst auf die Siedetemperatur von Gewebsflüssigkeit zu erwärmen, so dass sich Dampf bildet. Sobald eine ausreichende Erwärmung des Gewebes erreicht ist, geht das Gerät nach einem ersten Aspekt der Erfindung in eine zweite Etappe über. In dieser wird das Oszillieren des Gewebewiderstands veranlasst. In dieser Phase nimmt der Gewebewiderstand abwechseln hohe und niedrige Werte ein. Nachdem hohe Gewebewiderstände mit relativ trockenem Gewebe oder Dampfblasen im Gewebe, und niedrige Gewebewiderstände mit feuchtem Gewebe oder Gewebe ohne Dampfblasen assoziiert sind, oszilliert nicht nur der Gewebewiderstand, sondern es pulsiert auch der Gewebezustand, was eine pulsierende mechanische Beanspruchung des Gewebes ergeben kann. Außerdem kann, indem der elektrische Energieeintrag in das Gewebe periodisch reduziert wird, eine Feuchtigkeitserhöhung, Dampfbeseitigung oder Dampfverflüssigung (z.B. durch Kondensation oder Entweichen von Dampf) erreicht werden. Somit kann eine Widerstandsabsenkung des Gewebes bewirkt, und dadurch effektiv und/oder in der Summe ein erhöhter Gesamtenergieeintrag erzielt werden. Die periodische Reduktion des Gewebewiderstands bewirkt einen erhöhten Stromfluss zwischen den Elektroden im Vergleich zu Koagulationsprozessen, bei denen mit konstant hohem Gewebewiderstand gearbeitet wird.

Zur oszillierenden Steuerung des Gewebewiderstands ist eine Widerstands-Sollkurve festgelegt, wobei der Funktionsblock einen Regler für den Gewebewiderstand umfasst. Der Regler misst fortlaufend oder in einem engen Zeitraster den Gewebewiderstand und vergleicht diesen mit dem von der Gewebewiderstands-Sollkurve aktuell vorgegebenen Gewebewiderstand. Aus der sich ergebenden Abweichung bestimmt er die an das Gewebe anzulegende Spannung und liefert diese an die Koagulationselektroden, die somit eine hochfrequente Koagulationsspannung erhalten, die mit einer niedrigen Frequenz amplitudenmoduliert ist. Ihr Betrag oszilliert mit wenigen Hertz, vorzugsweise weniger als 30 Hz (oder weniger als 20 Hz), weiter vorzugsweise weniger als 20 Hz oder auch weniger als 10 Hz. bei vielen Fällen wird ein guter Effekt mit einer Oszillationsfrequenz zwischen 10 Hz und 20 Hz erreicht. Die Oszillationsfrequenz kann durch einen entsprechenden Funktionsblock fest vorgegeben sein oder von dem Funktionsblock, insbesondere auch in Abhängigkeit der Generatoreinstellung, des gefassten Gewebes und insbesondere in Abhängigkeit von dem Instrument, variabel vorgegeben werden. Die Oszillationsfrequenz kann somit unterschiedliche, situativ angepasste Werte annehmen.

Der Regler weist vorzugsweise eine Ausgangsspannungsbegrenzungseinrichtung auf, die während der Oszillation des Gewebewiderstands eine Maximalspannung und/oder eine Minimalspannung festlegt. Die Maximalspannung ist vorzugsweise so festgelegt, dass eine Funkenbildung während der Phasen hohen Gewebewiderstands oder eine sonstige thermische Schädigung des zu koagulierenden Gewebes wie auch umliegenden Gewebes unterbleibt. Z.B. kann die Maximalspannung auf einen Wert zwischen 80 V und 150 V, vorzugsweise 90 V bis 120 V festgelegt sein. Abweichende Werte sind möglich. Die Minimalspannung ist vorzugsweise auf einen von Null verschiedenen Wert festgelegt. Damit können eine zu schnelle Abkühlung des Gewebes und eine zu schnelle Kondensation von im Gewebe vorhandenem Dampf vermieden werden. Die Minimalspannung liegt z.B. in einem Bereich von 20 V bis 40 V.

Der Funktionsblock ist vorzugsweise darauf eingerichtet, die Widerstandsoszillation bei einem hohen Gewebewiderstand zu beenden, um dann in einer dritten Etappe in einen kontrollierten Abkühlvorgang überzugehen, der einen zweiten Aspekt der Erfindung bildet. In dem kontrollierten Abkühlvorgang erfolgt eine fortwährende Bestromung des Gewebes mit Spannung, deren Amplitude mit der Zeit abnimmt, wodurch sich die Abkühlung des Gewebes, die sonst sehr schnell abläuft, deutlich verlangsamt. Durch die fortlaufende Bestromung des zwischen den Elektroden gefassten Gewebes wird die Abkühlung desselben gegenüber der bei der Oszillation jeweils zu beobachtenden Zwischenabkühlung deutlich verlangsamt, wodurch der im Gewebe vorhandene Temperaturgradient verringert wird. Zonen die während des Abkühlvorgangs die für die Eiweißvernetzung optimale Temperatur durchlaufen, werden infolge des verminderten Temperaturgradienten im Gewebe vergrößert. Dadurch wird den beteiligten Eiweißen, insbesondere Kollagen, mehr Zeit und mehr Raum zur Ausbildung mechanisch fester, gegebenenfalls auch faseriger Strukturen gewährt. Eiweiße - insbesondere Kollagen - einander gegenüberliegender, aufeinander gedrückter Gefäßwände, können miteinander verschmelzen.

Obwohl der gebremste Abkühlvorgang zu einer verlängerten Abkühlzeitspanne führt, ist die Summe aus der für die Koagulation mit Widerstandsoszillation und die kontrollierte Abkühlung erforderlichen Zeit geringer, als sie bei Koagulation ohne Widerstandsoszillation und mit unkontrollierter Abkühlung erforderlich wäre. Damit ist eine Verkürzung der Zeitspanne für die Koagulation möglich, so dass insgesamt, einschließlich des Abkühlvorgangs, eine Behandlungszeit von weniger als 3s, insbesondere weniger als 2s erreicht werden kann. Obwohl die Zeit vom Schließen der Branchen des Instruments bis zum Wiederöffnen derselben derart kurz ist, kann eine sichere Gefäßversiegelung und somit ein qualitativ hochwertiges chirurgisches Ergebnis erzielt werden.

Zur Bestimmung des Verfahrensstatus und insbesondere zur Bestimmung der Anfangs- und Endzeitpunkte der verschiedenen Etappen kann der Funktionsblock wenigstens eine Gewebeeigenschaft überwachen. Eine solche Gewebeeigenschaft kann die am Gewebe anliegende Spannung, der durch das Gewebe fließende Strom, die applizierte Leistung, die auf das Gewebe übertragene Energiemenge, der Gewebewiderstand oder ähnliches sein. Z.B erfährt der Gewebewiderstand während der ersten Etappe zu Beginn der Bestromung typischerweise eine Phase der Abnahme, wonach er ein Minimum durchläuft und wieder ansteigt. Der Wiederanstieg ist mit Dampfbildung im oder am Gewebe verbunden. Nach einem ersten Aspekt der Erfindung werden nun in der zweiten Etappe des Koagulationsvorgangs Oszillationen des Gewebewiderstands erzeugt. Dies kann z.B. durch Vorgabe einer Sollkurve für den Gewebewiderstand erfolgen. Die an das Gewebe angelegte Koagulationsspannung wird mittels eines Reglers so bemessen, dass der Gewebewiderstand sich näherungsweise entsprechend der vorgegebenen Sollkurve verhält. Wenn die Oszillationen über eine vorbestimmte Zeitspanne hinweg (zum Beispiel etwa 0,5 s) stattgefunden haben oder wenn eine bestimmte Anzahl von Oszillationen (zum Beispiel 5 bis 15, vorzugsweise 7 bis 10, vorzugsweise 8) registriert worden ist, kann die dritte Etappe eingeleitet werden, die einen verlangsamten Abkühlvorgang bildet.

Die Widerstandsoszillationen und/oder die verlangsamte Abkühlung ermöglichen eine Fusion von Blutgefäßen mit erhöhter Sicherheit. Die Widerstandsoszillationen führen zu einem gepulsten Energieeintrag in das Gewebe mit periodischem Rückfeuchten des Gewebes durch Kondensation des isolierenden Dampfes und dadurch erhöhtem Energieeintrag. Es wird eine innige Verbindung der aneinander gepressten gegenüber liegenden Gefäßwände vorbereitet. Der verzögerte, d.h. verlangsamte Abkühlprozess erbringt dann eine gute Rekombination und Eiweißkettenbildung der beteiligten Eiweiße, insbesondere des Kollagens. Der verlangsamte Abkühlvorgang schafft vergrößerte Zonen in dem biologischen Gewebe, in denen während der Abkühlphase jeweils optimale Temperaturbedingungen für die Ausbildung von langen, vernetzten Eiweißketten erhalten werden.

Weitere Einzelheiten vorteilhafter Ausführungsformen des erfindungsgemäßen Generators sowie des erfindungsgemäßen Steuerverfahrens lassen sich der beigefügten Zeichnung, der Beschreibung sowie Unteransprüchen entnehmen. Es zeigen:
Figur 1 den Generator, ein angeschlossenes Instrument und ein zu fusionierendes Gefäß, in schematisierter Prinzipdarstellung,
Figur 2 ein zur Koagulation zwischen zwei Branchen gefasstes Gefäß, in schematisierter Schnittdarstellung,
Figur 3 den Generator, in schematisierter, ausschnittsweiser Funktionsblockdarstellung,
Figur 4 den zeitlichen Verlauf der von dem Generator abgegebenen Koagulationsspannung,
Figur 5 den zeitlichen Verlauf des in Reaktion auf die angelegte Koagulationsspannung entstehenden Gewebewiderstands,
Figur 6 die von dem Generator an das Gewebe abgegebene Leistung sowie die abgegebene Energie und
Figur 7 den von dem Instrument durch das Gewebe geleiteten Strom im zeitlichen Verlauf.

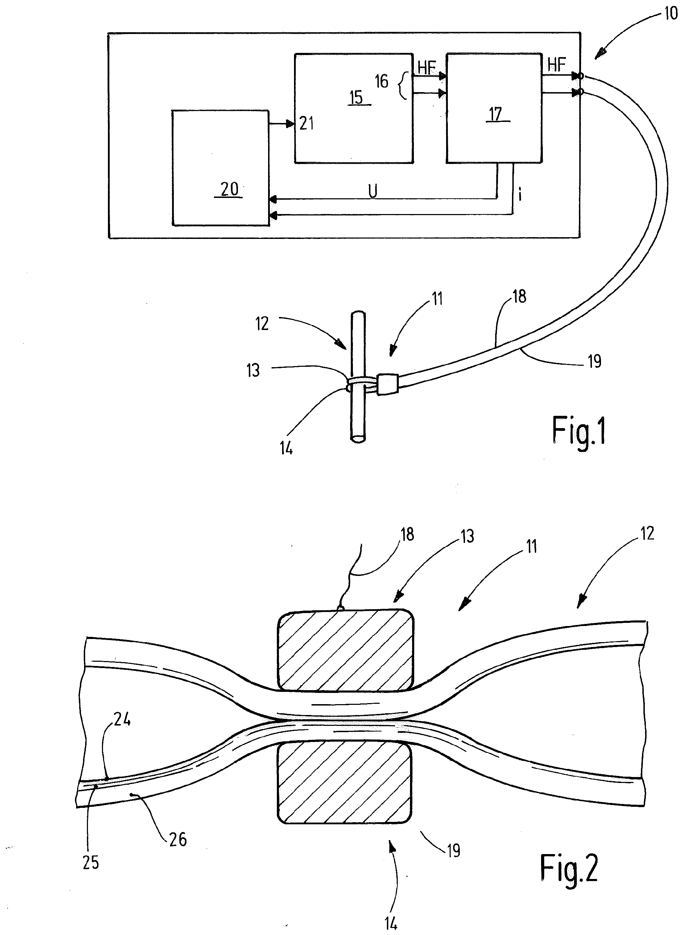

In Figur 1 sind ein Generator 10, ein von ihm versorgtes Gewebefusionsinstrument 11 sowie ein zu verschließendes Gefäß 12 sehr schematisiert veranschaulicht. Das Instrument 11 weist zwei Branchen 13, 14 auf, die zum Fassen des Blutgefäßes 12 dienen. Dazu vorgesehene Führungs- und Bedienungselemente, wie beispielsweise ein Handgriff mit Betätigungshebel, ein kürzerer oder längerer Schaft oder dergleichen, sind nicht veranschaulicht. Das Instrument 11 kann prinzipiell jede Bauform eines bekannten Gewebefusionsinstruments aufweisen, wie sie für die offenchirurgische oder laparoskopische Chirurgie in Anwendung ist.

Von den Branchen 13, 14 ist wenigstens eine beweglich, um das dazwischen gefasste Gefäß 12, wie es Figur 2 veranschaulicht, zusammendrücken zu können, so dass die Innenseiten der Gefäßwände aufeinander liegen und aneinander gedrückt werden. Außerdem kann das Instrument 11 ein bewegtes mechanisches Messer, ein Ultraschallmesser, ein bewegtes oder feststehendes mit Schneidspannung beaufschlagtes elektrisches Messer oder sonstig geartete Schneidelemente aufweisen. Die Erfindung betrifft das Gerät 10 und insoweit die Bestromung der Branchen 13, 14 und ist prinzipiell bei jedem Gewebe- oder Gefäßfusionsinstrument anwendbar, und zwar unabhängig davon, ob das Instrument keine, eine oder mehrere Schneidvorrichtungen zum Durchtrennen des fusionierten Elements aufweist.

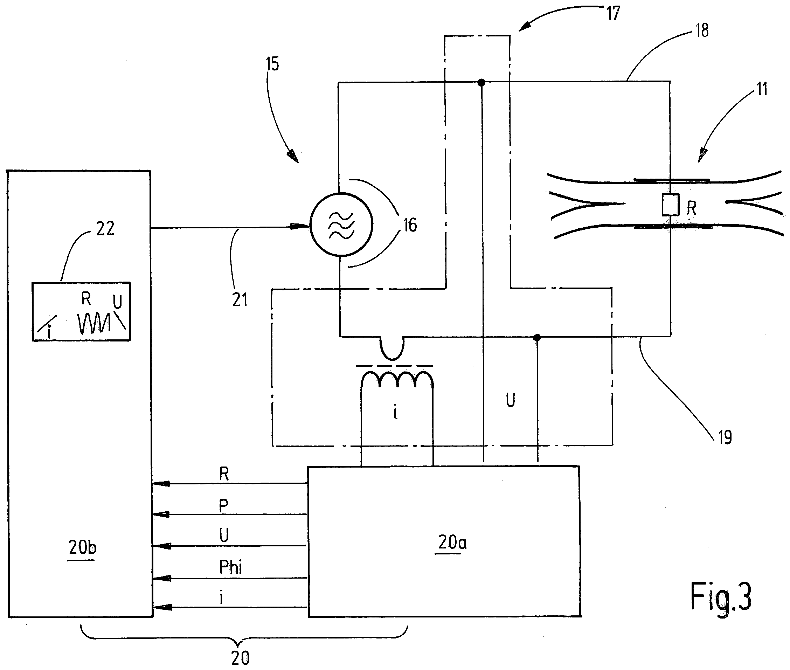

Das Gerät 10 weist einen Generator 15 auf, der an einem Ausgang 16 eine hochfrequente Koagulationsspannung HF bereitstellt, die gegebenenfalls über einen Sensorblock 17 und Leitungen 18, 19 zu dem Instrument 11 geleitet wird. Der Sensorblock 17 dient dazu, die Größe der von dem Generator 15 gelieferten HF-Spannung und/oder des von dem HF-Generator gelieferten HF-Stroms zu erfassen und als Signale u und/oder i an einen Funktionsblock 20 oder mehrere Funktionsblöcke 20a, 20b (siehe Figuren 1 und 3) zur Steuerung des Generators 15 zu liefern.

Der Generator 15 weist einen Eingang 21 auf, über den der Generator 15 Steuersignale empfangen kann. Diese können analoge oder digitale Signale sein, die die Höhe der von dem Generator 15 abgegebenen Koagulationsspannung u vorgeben. Die Steuersignale können von einem Funktionsblock 20 geliefert sein, der an den Sensorblock 17 angeschlossen ist, so dass er von diesem abgegebene Signale empfängt. Die Signale können z.B. Signale sein, die den von dem Generator 15 gelieferten HF-Strom und/oder die von dem Generator 15 bereitgestellte HF-Spannung kennzeichnen. Der Funktionsblock 20 kann in zwei oder mehrere Funktionsblöcke 20a, 20b unterteilt sein.

Der Funktionsblock 20 oder die Funktionsblöcke 20a, 20b können als gesonderte Bausteine oder als Programmbestandteil(e) eines Generatorsteuerungsprogramms oder durch jedes geeignete andere Mittel ausgebildet sein, mit dem sich der Betrieb des Generators 15 steuern lässt. Sie bilden einen Regler für eine etappenabhängig zu regelnde Größe, die z.B. der Strom i (z.B. in Etappe A), der Gewebewiderstand R (z.B. in Etappe B), der die Koagulationsspannung u (z.B. in Etappe C), oder die Leistung P (z.B. ebenfalls in Etappe C) sein kann. Der Regler kann darauf eingestellt sein, den Generator 15 in zumindest einer der Etappen innerhalb festgelegter, zeitlich konstanter oder einem gewünschten Zeitverlauf folgender Spannungsgrenzen zu steuern. Zusätzlich oder alternativ kann der Regler darauf eingestellt sein, den Generator 15 in zumindest einer der Etappen innerhalb festgelegter, zeitlich konstanter oder einem gewünschten Zeitverlauf folgender Stromgrenzen, Widerstandsgrenzen oder Leistungsgrenzen zu steuern. Die gewünschten Zeitverläufe können ansteigende oder absteigende Rampen oder andere stetige oder unstetige periodische oder nichtperiodische Funktionen sein.

Zur besseren Veranschaulichung der Struktur und Funktionsweise des Funktionsblocks 20 wird auf das Ausführungsbeispiel nach Figur 3 Bezug genommen. Der erste, an den Sensorblock 17 angeschlossene Funktionsblock 20a erfasst die von dem Generator 15 gelieferte, an den Branchen 18, 19 anliegende Koagulationsspannung u und den durch das Gefäß 12 oder sonstiges Gewebe fließenden Strom i. Die Größe des Stroms i hängt von der Größe der anliegenden Koagulationsspannung u und der Größe des Gewebewiderstands R ab, dessen Wert sich während der Koagulation des Gefäßes 12 oder sonstigen Gewebes ändert. Aus dem gemessenen Strom i und der Koagulationsspannung u kann der der Funktionsblock 20a bedarfsweise (zumindest näherungsweise) den vorhandenen Gewebewiderstand R=(u/i)*cos(Phi) und/oder die Leistung P=u*i*cos(Phi) und/oder die Phasenverschiebung Phi zwischen der Spannung u und dem Strom i berechnen und an den Funktionsblock 20b weitergeben. Außerdem kann der Funktionsblock 20a die erfasste Koagulationsspannung u und/oder den erfassten Strom i und/oder daraus berechnete Größen an den Funktionsblock 20b weitergeben.

Der Funktionsblock 20b empfängt, je nach Betriebsart oder Etappe des Koagulationsvorgangs, wenigstens eines der Signale, die den Gewebewiderstand R, die an das Gewebe übertragene Leistung P, die Phasenverschiebung Phi zwischen der Koagulationsspannung u und dem Strom i, die Koagulationsspannung u und/oder den Strom i kennzeichnen, der durch das Gewebe fließt. Der Funktionsblock-Teil 20b des Funktionsblocks 20 steuert den Koagulationsvorgang, indem er in jeder Etappe für mindestens eine der Größen R, P, Phi, u, i eine Sollkurve vorgibt, die z.B. in einem Speicher 22 bereitgehalten ist. Die Sollkurve kann mehrere Abschnitte enthalten, die jeweils für eine (oder mehrere) der Größen R, P, u, i gelten und den jeweiligen Sollwert für diese Größe vorgeben. Der oder die Sollwerte können je nach Art des angeschlossenen Instruments 11 oder nach Einstellung an einer Benutzerschnittstelle des Geräts 10 variieren.

Der Funktionsblock 20b enthält außerdem einen Regelblock, der die Differenz zwischen dem jeweiligen von der Sollkurve vorgegebenen Wert (R, P, Phi, u oder i) und dem von dem Funktionsblock 20a bestimmten Ist-Wert der jeweils kontrollierten Größe R, P, Phi, u oder i bestimmt. Aus dieser Differenz SOLL-IST wird innerhalb des Funktionsblocks 20b von dem Regelblock eine Sollspannung abgeleitet, die an den Generator 15 weitergegeben wird.

Die Steuerblöcke 20a, 20b können als Teil eines von einem Controller abgearbeiteten Programms ausgebildet sein, das wie folgt arbeitet und den Generator 15 wie folgt steuert:

Der Generator 15 ist in der Lage eine hochfrequente Koagulationsspannung im Bereich mehrerer 100 kHz, beispielsweise 350 kHz, abzugeben. Die von dem Generator 15 generierte Spannung kann z.B. im Bereich von 0 bis 150 V liegen. Andere Generatorspannungen sind anwendbar. Jedenfalls aber sind die Spannungen so bemessen, dass eine Funkenbildung zwischen den Branchen 13, 14 und dem biologischen Gewebe, beispielsweise dem Blutgefäß 12, unterbleibt. Außerdem ist der Generator 15 vorzugsweise so ausgebildet, dass er eine Leistung von zum Beispiel bis zu 120 W oder auch mehr erbringen kann. Weiter ist er vorzugsweise so ausgebildet, dass er einen HF-Strom von bis 2 A (oder darüber) liefern kann. Mit diesen Parametern ist der Generator 15 grundsätzlich zur Fusion des Blutgefäßes 12 oder eines sonstigen Gewebes, d.h. zum dauerhaften Verschließen desselben mittels des Instruments 11, geeignet.

Das Blutgefäß 12 weist ein Gefäßendothel 24 auf, das die innere Auskleidung, d.h. die Tunika interna des Blutgefäßes 12 bildet. Das Gefäßendothel besteht aus Endothelzellen, die ein einschichtiges Plattenepithel bilden, elastischen Fasern und Bindegewebe. Dieses sitzt auf einer mittleren auch als Tunika media bezeichneten Schicht 25, die aus Muskelzellen, Kollagenfasern, elastischen Fasern und Bindegewebe besteht. Die äußere auch als Tunika externa bezeichnete Schicht 26 besteht hauptsächlich aus Bindegewebe und elastischen Fasern. Insbesondere die Tunika media und die Tunika externa enthalten Kollagenfasern, die bei der Gewebefusion miteinander zu verschmelzen sind.

Zur Durchführung des Gewebefusionsprozesses wird zunächst das Gefäß 12 zwischen den Branchen 13, 14 gefasst und gemäß Figur 2 zusammengedrückt, so dass sich die einander gegenüber liegenden Innenflächen der Gefäßwände berühren, das Blut zwischen den Branchen 13, 14 herausgedrückt wird und das Gefäß 12 vollständig abgeklemmt ist. Die Branchen 13, 14 üben dabei einen Anpressdruck auf das Gefäß 12 aus.

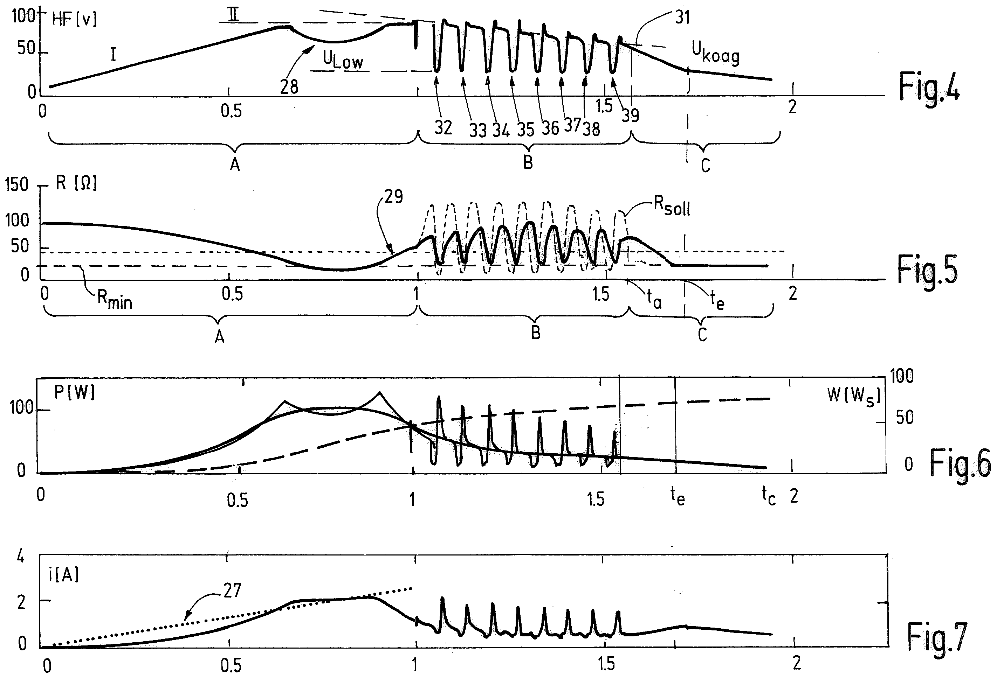

Der Fusionsprozess beginnt gemäß Figur 4 bis 7 mit einer ersten Etappe, die vorzugsweise höchstens etwa 1 s andauert und innerhalb derer das Gefäß 12 zwischen den Branchen 13, 14 durch Stromdurchfluss erwärmt wird. Programmseitig kann die Etappe A auf eine festgelegte Zeitspanne, z.B. 2,5 s begrenzt sein. Beispielsweise wird in Etappe A der durch das Gewebe zu schickende Strom i als Rampe, z.B. zeitlich linear ansteigend, vorgegeben, wie in Figur 7 mit einer gepunkteten Linie 27 dargestellt ist. Der Funktionsblock 20a erfasst in diesem Fall den Strom i und gibt diesen an den Funktionsblock 20b, der als Regler arbeitet. Der Funktionsblock 20b generiert eine Stellgröße für den Generator 15 und gibt diese an dessen Eingang 21. Zugleich kann der Funktionsblock 20b aber auch die an die Branchen 13, 14 angelegte Koagulationsspannung HF berücksichtigen und z.B. nach einer zeitabhängigen Funktion, z.B. einer Spannungsrampe I (Figur 4) begrenzen, z.B. um schädliche Effekte an dem Gewebe zu verhindern. Die Begrenzung kann z.B. zunächst gemäß einer vorgegebenen zeitabhängigen Funktion, beispielsweise der in Figur 4 gestellten linearen Rampe I, und optional außerdem durch eine Maximalspannung II begrenzt werden. Durch die Spannungsbegrenzung bleibt der Strom i eine gewisse Zeit (z.B. etwa 0,6 s) unter der Sollkurve 27.

Gleichzeitig mit der Stromzunahme nimmt der Gewebewiderstand R, der in Figur 5 dargestellt ist, mit fortschreitender Erwärmung des Gewebes durch Freisetzen von Gewebeflüssigkeit und steigende Mobilität der gelösten Ionen ab. Mit abnehmendem Widerstand steigt der Strom, so dass der Stromregler die Spannung absenkt. Sie Fällt unter die Begrenzung, so dass der Strom i der Vorgabe 27 nun folgt.

Alternativ zu dieser Art der Steuerung kann auch die Koagulationsspannung u nach einer vorgegebenen Zeitfunktion, z.B. als Rampe, in Stufen oder ähnlich gesteuert werden. Dies kann der Funktionsblock 20b über die sich zeitlich entsprechend verlaufende Sollspannung vorgeben.

In der ersten Etappe fällt der Widerstand R des Gewebes mit ansteigendem Strom und zunehmender Erwärmung zunächst auf einen Wert Rₘᵢₙ, der typischerweise unter 50 Ohm liegt. Aufgrund der zunehmenden Gewebetemperatur fällt der ohmsche Widerstand R des biologischen Gewebes auf sehr geringe Werte, wie beispielsweise kaum mehr als 20 Ohm ab, in vielen _Fällen sogar unter 10, 5 oder 2 Ohm. Mit zunehmender Gewebeerwärmung und beginnender Dampfbildung erfolgt ein Wiederanstieg des Gewebewiderstands R, wie es in Figur 5 an der Stelle 29 zu erkennen ist. Dieser Zeitpunkt ist etwa nach 1s erreicht. Während dieses Vorgangs ist die auf das Gewebe übertragene Leistung relativ hoch, wie es Figur 6 zeigt. Erreicht oder überschreitet der Gewebewiderstand R einen Grenzwert von z.B. 50 Ohm und/oder ein vielfaches von Rₘᵢₙ und/oder eine bestimmte Phasenverschiebung Phi zwischen der Spannung u und dem Strom i ist die Etappe A beendet.

Zu diesem Zeitpunkt (etwa bei 1s) hat die auf das Gefäß 12 übertragene Leistung P ihr Maximum überschritten und nimmt wegen des zunehmenden Gewebewiderstands infolge zunehmender Dampfbildung bzw. Gewebeaustrocknung ab. Die auf das Gefäß 12 übertragene Energie W hat bei dem vorliegenden Ausführungsbeispiel zu diesem Zeitpunkt etwa 50 J erreicht. Es können alternativ andere Energiewerte vorgesehen sein.

Der Funktionsblock 20b kann so ausgebildet sein, dass er den Verfahrensstatus anhand der verstrichenen Zeit, alternativ anhand der auf das Gefäß 12 übertragenen elektrischen Energie W, alternativ anhand der Größe und/oder des zeitlichen Verlaufs des Gewebewiderstands R oder weiter alternativ anhand der Größe und/oder des zeitlichen Verlaufs des Stroms i bestimmt. Ein charakteristischer Wert für den Verfahrensstatus ist das beginnende Sieden der Gewebsflüssigkeit, was damit einhergeht, dass zumindest Teile des Gefäßes 12 Siedetemperatur erreicht haben. Überwacht der Funktionsblock 20b den Strom, kann dies durch den Funktionsblock 20b anhand des Stromverlaufs 27 erkannt werden. Überwacht der Funktionsblock 20b die auf das Gefäß 12 übertragene Energie W, kann der Funktionsblock 20b das beginnende Sieden von Gewebsflüssigkeit durch Erreichen einer bestimmten auf das Gefäß 12 übertragenen Energiemenge (zum Beispiel 50 Wattsekunden) erkennen. Überwacht der Funktionsblock 20 den Gewebewiderstand R, kann er das beginnende Sieden von Gewebsflüssigkeit durch Überschreiten einer Widerstandsgrenze von zum Beispiel 42,5 Ohm nach Durchlaufen eines Widerstandsminimums erkennen.

Unabhängig davon, welche der genannten Größen von dem Funktionsblock 20, 20b überwacht wird, erkennt dieser als Funktionsstatus das Ende der Etappe A (z.B. durch das beginnende Sieden von Gewebsflüssigkeit anhand des Widerstands R) und steuert nun den Generator 15 in eine Gewebsfusionsphase, die vorzugsweise etwa eine halbe Sekunde oder geringfügig länger dauert. Während dieser Phase kann der Generator 15 von dem Funktionsblock 20b so gesteuert werden, dass er die Koagulationsspannung u oder den Gewebewiderstand R mit zeitlich gemäß einer vorgegebenen Funktion 31 verlaufendem Sollwert und periodischen Spannungseinbrüchen bzw. Spannungsabsenkungen 32 bis 39 erzeugt. Der vorgegebene Maximalwert der Koagulationsspannung u kann zeitlich konstant sein oder, wie es Figur 4 nahelegt, einer abfallenden, zeitlich veränderlichen Funktion, z.B. einer abfallenden Geraden folgen. Andere Spannungsverläufe, z.B. in Gestalt einer abfallenden e-Funktion oder auch ein ansteigender Spannungsverlauf sind nutzbar.

In der Etappe B arbeitet der Funktionsblock 20b vorzugsweise als Regler für den Gewebewiderstand R. Dazu gibt der Speicher 22 eine Wunsch-Zeitfunktion für den gewünschten Gewebewiderstand Rₛₒₗₗ vor und liefert diese Funktion an den Regelblock. Diese gewünschte Zeitfunktion ist beispielsweise eine periodische zeitabhängige Funktion, z.B. eine Sinusfunktion, die in Figur 5 gestrichelt dargestellt ist. Der Funktionsblock 20a ermittelt den tatsächlichen Gewebewiderstand Rᵢₛₜ liefert diesen ebenfalls an den Regelblock. Dieser steuert dementsprechend den Generator 15 unter Beachtung der Spannungsgrenze II, die die von dem Generator 15 maximal abgebbare Spannung noch immer auf einen Maximalwert, z.B. 90 V bis 120 V begrenzt. Außerdem begrenzt der Funktionsblock 20b entweder die Koagulationsspannung u nach unten, so dass sie einen Minimalwert von z.B. 25 V nicht unterschreitet. Mit der Spannungsbegrenzung nach oben lassen sich Funkenüberschläge an dem Gewebe oder sonstige thermische Gewebeschäden vermeiden. Mit der Spannungsbegrenzung nach unten wird eine zu schnelle oder eine übermäßige Kondensation von Dampf vermieden.

Infolge der Regelung des Gewebewiderstands treten gemäß Figur 4 Spannungsabsenkungen 32 bis 39 auf, bei denen der Generator 15 jeweils kurz seiner Leistung reduziert, so dass die abgegebene Spannung von einem Wert von etwa 90 V bis 120 V auf einen Minimalwert von zum Beispiel 20 V oder 25 V abfällt. Jeweils bei Spannungsabsenkung fällt zunächst auch die auf das Gefäß 12 übertragene Leistung ab, wie Figur 6 zeigt. Durch den zugleich abfallenden Gewebewiderstand wird jedoch bei der periodisch jeweils nachfolgenden Spannungserhöhung eine erhöhte Leistung auf das Gewebe übertragen. Im Mittel ist die auf das Gewebe übertragene Leistung bei periodischer Absenkung des Gewebewiderstands jedoch größer, als wenn die Koagulation ganz mit hoher Koagulationsspannung u von z.B. 100 V und somit konstant hohem Gewebewiderstand erfolgen würde.

Durch die periodische Widerstandsmodulation kann bereits erzeugter Dampf und/oder Gewebe des Gefäßes 12 rückfeuchten. Es ergeben sich entsprechend den in Figur 5 veranschaulichten Widerstandsoszillationen und nachfolgenden Leistungsspitzen gemäß Figur 6 Druckpulsationen, die dazu beitragen können, das Gefäßendothel 24 zu durchdringen. Damit können Eiweißbestandteile der Tunika media 25 und gegebenenfalls auch der Tunika externa 26 einander gegenüber liegender Gefäßwände Kontakt zueinander erhalten und miteinander verschmelzen.

Der Funktionsblock 20 kann den Verfahrensstatus des Gefäßes 12 zum Beispiel anhand einer Zeitkurve für den Verlauf des Gewebewiderstands mit einer fest oder variabel vorgegebenen Anzahl von Oszillationen des Gewebewiderstands R oder Spannungsabsenkungen 32 bis 39 vorgeben. Alternativ ist es auch möglich, die Oszillationen mit Amplitude und Frequenz vorzugeben und die Anzahl der Spannungsabsenkungen 32 bis 39 oder der Widerstandsoszillationen zu erfassen. Dazu können die vorgenommenen Spannungsverminderungen abgezählt und, wenn eine Grenze von zum Beispiel 8 oder 9 erreicht ist, die Etappe B beendet werden. Die Etappe B endet jedenfalls bei einem hohen Gewebewiderstand und somit auch bei einer hohen (nicht abgesenkten) Koagulationsspannung u. Ähnlich kann der Funktionsblock 20 in einer weiter abgewandelten Ausführungsform die Leistungsmaxima und/oder Leistungsminima gemäß Figur 6 oder die Stromspitzen nach Figur 7 überwachen und zählen, um den Verfahrensstatus zu erfassen und das Ende der Etappe B zu erkennen.

Stellt der Funktionsblock 20 auf irgendeine der vorbeschriebenen Weisen fest, dass die zweite Etappe B beendet ist, wechselt der Funktionsblock 20 die Ansteuerung des Generators 15 so, dass dieser in eine kontrollierte Gewebeabkühlphase Etappe C eintritt. In dieser wird dem Gefäß 12 weiterhin Strom zugeführt, um die Gewebeabkühlung gezielt zu verlangsamen. Damit sinkt der Gewebewiderstand R gemäß Figur 5 in dieser zu einem Zeitpunkt tₐ beginnenden Phase weniger steil ab, als während der Koagulationsphase bei den Spannungsabsenkungen 32 bis 39. Dies wird erreicht, indem beispielsweise die Koagulationsspannung u gemäß Vorgabe eines zeitlichen Spannungsverlaufs geführt wird, der in dem Speicher 22 abgelegt ist. Der Funktionsblock 20b gibt die Koagulationsspannung gemäß einer zeitabhängigen Funktion, z.B. als abfallende Rampe vor. Die vorgegebene Spannung kann als Steuersignal entweder direkt an den Generator oder alternativ an den Regelblock gegeben werden, der andererseits die tatsächlich von dem Generator 15 abgegebenen Koagulationsspannung u erhält und den Generator anhand der gebildeten Differenz steuert.

Die Koagulationsspannung u wird vorzugsweise mit einer vorgegebenen Rate von beispielsweise -200 V/s abgesenkt. Es können auch andere Absenkgeschwindigkeiten (zum Beispiel -150 V/s oder -250 V/s) angewendet werden. Außerdem kann die Absenkgeschwindigkeit während der Abkühlphase, falls gewünscht, variiert werden.

Während der Abkühlphase tritt eine Rückfeuchtung des Gewebes auf, wobei das Gewebe zonenweise verschiedene Temperaturbereiche ausgehend von etwa 150°C bis 170°C abkühlend durchläuft. Damit geht ein Absinken des Gewebewiderstands R einher, das jedoch aufgrund der weiteren Bestromung deutlich langsamer ist, als die Widerstandsabsenkungen während der Widerstandsoszillationen in der Etappe B. Dadurch wird der Temperaturgradient im biologischen Gewebe durch die verlangsamte Abkühlung im Vergleich zu einer ungebremsten Abkühlung vermindert. Es bilden sich relativ großvolumige Zonen mit verlängerter Bestandsdauer aus, deren Temperatur in einem Temperaturfenster liegt, das für die Eiweißverkettung günstig ist. Damit steht für jeden Punkt des betroffenen Gewebes mehr Zeit für die Ausbildung mechanisch haltbarer Eiweißstrukturen zur Verfügung.

Mit zunehmendem Abbau bzw. Verflüchtigung des Dampfes nimmt der Gewebewiderstand R unter einen Grenzwert ab. Dieser Grenzwert kann ein vorgegebener Grenzwert oder alternativ ein Grenzwert sein, der sich aus dem Gewebewiderstand während der Widerstandsoszillationen oder aus dem Widerstandsverlauf aus der Etappe A ergibt. Z.B. fällt der Gewebewiderstand in der Etappe B wegen der nicht unterschreitbaren Minimalspannung des Generators 15 nicht ganz so weit ab, wie es die Widerstands-Sollkurve vorgibt. Der auftretende minimale Gewebewiderstand Rₘᵢₙ kann jedoch registriert werden. Erreicht der Gewebewiderstand R den registrierten minimalen Gewebewiderstand Rₘᵢₙ oder ein festgelegtes Vielfaches davon (z.B. 1,5*Rmin), kann dies als Ereignis zur Beendigung der spannungsgeführten Abkühlphase der Etappe C genutzt werden. Der Funktionsblock 20 schaltet zu diesem Zeitpunkt tₑ auf Leistungsregelung um. Dem Gewebe wird nun eine solche Koagulationsspannung u zugeführt, dass die Leistung P, wie Figur 6 zeigt, nach dem Zeitpunkt tₑ weiter, zum Beispiel linear oder auch nach einer vorgegebenen anderen Kurve, abnimmt. Das Ende der Koagulation und somit das Abschalten des Generators 15 veranlasst der Funktionsblock 20 bzw. 20b dann beispielsweise zeitgesteuert und/oder nach Erreichen einer bestimmten Energiemenge W und/oder bei Erreichen einer bestimmten Leistung oder nach sonstigen Kriterien zu einem Zeitpunkt t_{c}.

Der Grenzwert des Widerstands kann alternativ auch z.B. derjenige Wert Rₘᵢₙ sein, den der Gewebewiderstand als Minimum vor seinem Anstieg 28 erreicht und/oder derjenige Wert, auf den der Gewebewiderstand während der Spannungsabsenkungen 32 - 39 abfällt. Der Funktionsblock 20, 20b kann diesen Wert bestimmen und abspeichern, um ihn dann zur Erkennung des Endes der Abkühlphase als Grenzwert zu nutzen.

Das erfindungsgemäße Gerät 10 und das erfindungsgemäße Verfahrenskonzept gestattet jeweils eine besonders schnelle, schonende und sichere Fusion von Gefäßen 12 zwischen zwei Koagulationselektroden 13, 14. Dabei werden in dem biologischen Gewebe Widerstandsoszillationen erzeugt, bei denen jeweils alternierend ein Gewebewiderstandswert von z.B. 50 Ohm über- und unterschritten wird. Danach wird eine Phase verlangsamter Gewebeabkühlung durchlaufen, während derer das Gewebe 12 mit vorzugsweise zeitlich abnehmender Koagulationsspannung bestromt wird, um einen gegenüber der sofortigen Spannungsabschaltung wesentlich verlangsamten Abkühlprozess zu erreichen. Damit werden einerseits eine gute Fusion des Kollagens der aneinander gepressten Gefäßwände und andererseits eine mechanisch stabile Verfestigung des Kollagens erreicht. Durch die genannte Prozessführung ist die erforderliche Fusionszeit gegenüber herkömmlichen Verfahren verkürzt, die unerwünschte Schädigung von umliegenden Gewebe durch die verkürzte Einwirkzeit des hochfrequenten Stroms reduziert und der Gefäßverschluss sicherer.

Bezugszeichen:

| | |
|---|---|
| 10 | Gerät |
| 11 | Instrument |
| 12 | Blutgefäß |
| 13, 14 | Branchen |
| 15 | Generator |
| HF | von dem Generator 15 abgegebene Koagulationsspannung |
| 16 | Ausgang des Generators 15 |
| 17 | Sensorblock |
| 18, 19 | Leitungen |
| u | die Koagulationsspannung HF kennzeichnendes Signal |
| i | den Strom des Generators 15 kennzeichnendes Signal |
| R | Gewebewiderstand |
| P | auf das Gewebe übertragene Leistung |
| Phi | Phasenwinkel zwischen u und i |
| 20 | Funktionsblock/Regler |
| 21 | Eingang zur Steuerung des Generators 15 |
| I | Rampe zur Spannungsbegrenzung |
| II | Spannungsgrenze |
| 22 | Speicher |
| 24 | Gefäßendothel (Tunica Interna) |
| 25 | Tunica Media |
| 26 | Tunica Externa |
| 27 | Vorgabe für den Soll-Strom |
| 28 | Abschnitt abfallender Spannung u |
| 29 | Wideranstieg des Gewebewiderstands |
| W | auf das Gefäß 12 übertragene Energie |
| | |
| 32 - 39 | Spannungsabsenkungen |
| tₐ, tₑ | Anfang und Ende der spannungsgeführten Abkühlphase |
| t_{c} | Ende der Bestromung |

## Patentansprüche

1. Gerät (10) zur Versorgung eines Gewebefusionsinstruments, insbesondere eines Instruments (11) zur Gefäßfusion,
mit einem Generator (15) zur Erzeugung einer Koagulationsspannung (u) zum Betrieb des Instruments (11), mittels dessen die Koagulationsspannung (u) an zu fusionierendes Gewebe (12) zur Aufheizung desselben wenigstens auf Siedetemperatur von Gewebsflüssigkeit als Koagulationsspannung anzulegen ist, wobei der Generator (15) mindestens hinsichtlich der Größe der erzeugten Koagulationsspannung (u) steuerbar ist,
mit einem Funktionsblock (20) zur Steuerung des Generators (15), der darauf eingerichtet ist, die Koagulationsspannung (u) mit niedriger Frequenz amplitudenmoduliert zu steuern, so dass ein Oszillieren des Gewebewiderstands (R) veranlasst wird und dieser abwechseln hohe und niedrige Werte einnimmt, wobei der Funktionsblock (20) eine Ausgangsspannungsbegrenzungseinrichtung aufweist, die während der Oszillation des Gewebewiderstands (R) eine Maximalspannung festlegt, die so festgelegt ist, dass eine Funkenbildung während der Phasen hohen Gewebewiderstands (R) unterbleibt, **dadurch gekennzeichnet, dass** zur oszillierenden Steuerung des Gewebewiderstands (R) eine Widerstands-Sollkurve festgelegt ist und dass der Funktionsblock einen Regler für den Gewebewiderstand (R) umfasst,
wobei der Regler dazu eingerichtet ist, fortlaufend oder in einem engen Zeitraster den Gewebewiderstand zu messen und diesen mit dem von der Gewebewiderstands-Sollkurve aktuell vorgegebenen Gewebewiderstand zu vergleichen sowie aus der sich ergebenden Abweichung die an das Gewebe anzulegende Spannung zu bestimmen und diese an die Koagulationselektroden zu liefern.

2. Gerät nach Anspruch 1, **dadurch gekennzeichnet, dass** der Regler eine Ausgangsspannungsbegrenzungseinrichtung aufweist, die während der Oszillation des Gewebewiderstands eine Maximalspannung und/oder eine Minimalspannung festlegt.

3. Gerät nach einem der vorstehenden Ansprüche, **dadurch gekennzeichnet, dass** der Funktionsblock (20) darauf eingerichtet ist, die Widerstandsoszillation bei hohem Gewebewiderstand zu beenden.

4. Gerät (10) zur Versorgung eines Gewebefusionsinstruments mit zwei Koagulationselektroden (13, 14),
mit einem Generator (15) zur Erzeugung einer Koagulationsspannung (u) zum Betrieb des Instruments (11), mittels dessen die Koagulationsspannung (u) über die beiden Koagulationselektroden (13, 14) an ein zu fusionierendes, zwischen den Koagulationselektroden (13, 14) gehaltenes Gefäß (12) anzulegen ist, um dieses auf Siedetemperatur von Gewebsflüssigkeit aufzuheizen und bei aufeinander gepressten Gefäßwänden zu fusionieren, wobei der Generator (15) mindestens hinsichtlich der Größe der erzeugten Koagulationsspannung (u) steuerbar ist,
mit einem Funktionsblock (20) zur Steuerung des Generators (15), zur Durchführung eines Gewebeabkühlvorgangs unter fortlaufender Bestromung des Instruments (11) bei zwischen den Elektroden (13, 14) zusammengepressten Gefäßwänden mit Koagulationsspannung (u) zeitlich abnehmender Amplitude,
**dadurch gekennzeichnet,**
**dass** der Funktionsblock (20) darauf eingerichtet ist, die Koagulationsspannung (u) nach einer vorgegebenen Kurve zu reduzieren.

5. Gerät nach Anspruch 4, **dadurch gekennzeichnet, dass** der Funktionsblock (20) darauf eingerichtet ist, die Koagulationsspannung (u) während des Gewebeabkühlvorgangs fortlaufend oder in mehreren Stufen zu vermindern.

6. Gerät nach einem der Ansprüche 4 oder 5, **dadurch gekennzeichnet, dass** der Funktionsblock (20) zusätzlich oder alternativ hinsichtlich der Größe der abgegebenen Leistung (P) steuerbar und dazu eingerichtet ist, während des Gewebeabkühlvorgangs den Verfahrensstatus weiter zu überwachen und anhand desselben von der Bestromung mit abnehmender Koagulationsspannung (u) zu einer Bestromung mit geregelter Leistung (P) überzugehen.

7. Gerät nach einem der Ansprüche 1 bis 3 und nach einem der Ansprüche 4 bis 6.

## Claims

1. A device (10) for supplying a tissue fusion instrument, in particular an instrument (11) for tissue fusion, comprising a generator (15) for generating a coagulation voltage (u) for operating the instrument (11), by means of which the coagulation voltage (u) is to be applied as the coagulation voltage to a tissue (12) to be fused for heating said tissue at least to the boiling temperature of tissue fluid, wherein the generator (15) is controllable at least with regard to the magnitude of the generated coagulation voltage (u),
comprising a function block (20) for controlling the generator (15), which is configured to control the coagulation voltage (u) with low frequency in an amplitude-modulated manner, so that an oscillation of the tissue resistance (R) is caused and this alternately assumes high and low values,
wherein the function block (20) has an output voltage limiting device which sets a maximum voltage during the oscillation of the tissue resistance (R), which is set such that sparking is avoided during the phases of high tissue resistance (R),
**characterized in that** a nominal resistance curve is defined for the oscillating control of the tissue resistance (R) and that the function block comprises a controller for the tissue resistance (R),
wherein the controller is configured to measure the tissue resistance continuously or in a tight time frame and to compare this to the tissue resistance currently specified by the nominal tissue resistance curve and to determine the voltage to be applied to the tissue from the resulting deviation and to deliver this voltage to the coagulation electrodes.

2. The device according to claim 1, **characterized in that** the controller comprises an output voltage limiting device that sets a maximum voltage and/or a minimum voltage during the oscillation of the tissue resistance.

3. The device according to one of the preceding claims, **characterized in that** the function block (20) is configured to terminate the resistance oscillation at a high tissue resistance.

4. A device (10) for supplying a tissue fusion instrument having two coagulation electrodes (13, 14),
comprising a generator (15) for generating a coagulation voltage (u) for operating the instrument (11), by means of which the coagulation voltage (u) is to be applied via the two coagulation electrodes (13, 14) to a vessel (12) to be fused, which is held between the coagulation electrodes (13, 14), in order to heat it to a boiling temperature of tissue fluid and to fuse it when the vessel walls are pressed against one another, wherein the generator (15) is controllable at least with regard to the magnitude of the coagulation voltage (u) generated,
comprising a function block (20) for controlling the generator (15) for performing a tissue cooling process under continuous current application to the instrument (11), when the vessel walls are pressed together between the electrodes (13, 14), with a coagulation voltage (u) of decreasing amplitude over time,
**characterized in that**
the function block (20) is configured to reduce the coagulation voltage (u) according to a predetermined curve.

5. The device according to claim 4, **characterized in that** the function block (20) is configured to reduce the coagulation voltage (u) continuously or in multiple steps during the tissue cooling process.

6. The device according to one of claims 4 or 5, **characterized in that** the function block (20) is additionally or alternatively controllable with respect to the magnitude of the output power (P) and is configured to continue monitoring the process status during the tissue cooling process and, based on the latter, to transition from current application with decreasing coagulation voltage (u) to current application with controlled power (P).

7. The device according to one of claims 1 to 3 and according to one of claims 4 to 6.

## Revendications

1. Appareil (10) destiné à l'alimentation d'un instrument de fusion de tissus, notamment d'un instrument (11) de fusion de vaisseaux,
comprenant un générateur (15) pour générer une tension de coagulation (u) pour le fonctionnement de l'instrument (11) à l'aide duquel la tension de coagulation (u) doit être appliquée en tant que tension de coagulation aux tissus (12) à fusionner, en vue de chauffer ceux-ci jusqu'à la température d'ébullition du liquide tissulaire, le générateur (15) pouvant être commandé au moins concernant la valeur de la tension de coagulation (u) produite,
comprenant un bloc fonctionnel (20) pour commander le générateur (15), qui est conçu pour contrôler la tension de coagulation (u) par modulation d'amplitude avec une faible fréquence, de manière à provoquer une oscillation de la résistance tissulaire (R) et faire en sorte que celle-ci adopte en alternance des valeurs élevées et faibles,
le bloc fonctionnel (20) présentant un dispositif de limitation de tension de sortie qui définit, pendant l'oscillation de la résistance tissulaire (R), une tension maximale qui est fixée de manière à éviter la formation d'étincelles pendant les phases de résistance tissulaire (R) élevée,
**caractérisé en ce que** pour la commande oscillante de la résistance tissulaire (R), une courbe de consigne de résistance est définie, et **en ce que** le bloc fonctionnel comprend un régulateur pour la résistance tissulaire (R),
le régulateur étant conçu pour mesurer la résistance tissulaire en continu ou dans une trame de temps serrée et pour la comparer avec la résistance tissulaire actuellement prédéfinie par la courbe de consigne de résistance tissulaire, et pour déterminer, à partir de l'écart obtenu, la tension devant être appliquée aux tissus et la transmettre aux électrodes de coagulation.

2. Appareil selon la revendication 1, **caractérisé en ce que** le régulateur présente un dispositif de limitation de tension de sortie qui définit une tension maximale et/ou une tension minimale pendant l'oscillation de la résistance tissulaire.

3. Appareil selon une des revendications précédentes, **caractérisé en ce que** le bloc fonctionnel (20) est conçu pour mettre fin à l'oscillation de la résistance en cas de résistance tissulaire élevée.

4. Appareil (10) destiné à l'alimentation d'un instrument de fusion de tissus avec deux électrodes de coagulation (13, 14),
comprenant un générateur (15) pour générer une tension de coagulation (u) pour le fonctionnement de l'instrument (11) à l'aide duquel la tension de coagulation (u) doit être appliquée, par l'intermédiaire des deux électrodes de coagulation (13, 14), à un vaisseau (12) à fusionner qui est maintenu entre les électrodes de coagulation (13, 14), en vue de le chauffer jusqu'à la température d'ébullition du liquide tissulaire et de le fusionner en pressant les unes contre les autres les parois de vaisseau, le générateur (15) pouvant être commandé au moins concernant la valeur de la tension de coagulation (u) produite,
comprenant un bloc fonctionnel (20) pour commander le générateur (15), aux fins de réaliser une opération de refroidissement des tissus par alimentation continue de l'instrument (11) avec du courant, lorsque les parois de vaisseau sont pressées entre les électrodes (13, 14), avec une tension de coagulation (u) à amplitude diminuant dans le temps,
**caractérisé en ce que**
le bloc fonctionnel (20) est conçu pour réduire la tension de coagulation (u) suivant une courbe prédéterminée.

5. Appareil selon la revendication 4, **caractérisé en ce que** le bloc fonctionnel (20) est conçu pour faire diminuer la tension de coagulation (u) pendant l'opération de refroidissement des tissus, de manière continue ou en plusieurs étapes.

6. Appareil selon une des revendications 4 ou 5, **caractérisé en ce que** le bloc fonctionnel (20) peut en plus ou de manière alternative être commandé en ce qui concerne la valeur de la puissance (P) délivrée et est conçu pour continuer à surveiller le statut du procédé pendant l'opération de refroidissement des tissus et, sur la base dudit statut, passer de l'alimentation en courant avec une tension de coagulation (u) qui diminue, à une alimentation en courant avec une puissance (P) régulée.

7. Appareil selon une des revendications 1 à 3 et selon une des revendications 4 à 6.
